# EUROPEAN PATENT SPECIFICATION

(11) **EP 2 512 348 B1**
(45) Date of publication and mention of the grant of the patent: **21.10.2015**
(21) Application number: 10776129.8
(22) Date of filing: 10.11.2010
(51) Int. Cl.: A61B 17/00, B25G 3/18, A61B 19/00

(54) **ELASTOMERIC GRIP FOR A SURGICAL INSTRUMENT**
ELASTOMERGRIFF FÜR EIN CHIRURGISCHES INSTRUMENT
POIGNÉE EN ÉLASTOMÈRE POUR INSTRUMENT CHIRURGICAL

(30) Priority: 16.12.2009 GB 0921940
(43) Date of publication of application: 24.10.2012
(73) Proprietor: DePuy (Ireland), Co Cork (IE)
(72) Inventor: BEEDALL, Duncan, Yorkshire LS11 8DT (GB); MEGGETT, Philip, Yorkshire LS11 8DT (GB); YOUNG, Duncan, Yorkshire LS11 8DT (GB)
(74) Representative: Burton, Nick
(86) International application number: PCT/GB2010/051869
(87) International publication number: WO 2011/073632

(56) References cited:
- DE-A1-102007 014 737
- US-A- 5 653 721
- US-A1- 2009 012 351
- US-B1- 6 358 267

## Description

The present invention relates to a surgical instrument. The present invention further relates to a method of manufacturing a surgical instrument.

It is known to provide surgical instruments comprising two or more components. As a first example, a surgical instrument set may be provided in which there is a single handle arranged to couple to a range of other components, for instance a reamer or a positioning tool. The term "surgical instrument" as used herein is intended to refer either to a whole assembled surgical instrument, for instance a handle coupled to a reamer, or to a part of a surgical instrument. For instance, the first component of the surgical instrument may comprise the handle a second component may comprise a clip or latch coupled to the handle and arranged to move relative to the handle to secure the handle to a further component or a further surgical instrument.

As a second example, a first component of a surgical instrument could comprise a handle and a second component could comprise part of an adjustment mechanism, for instance for extending the length of the handle.

For a surgical instrument comprising a handle and a clip arranged to secure the handle to a further component, it is known for the clip to be pivotally coupled to the handle and for a spring to be provided coupled between the handle and the clip and arrange to bias part of the clip away from or towards the handle.

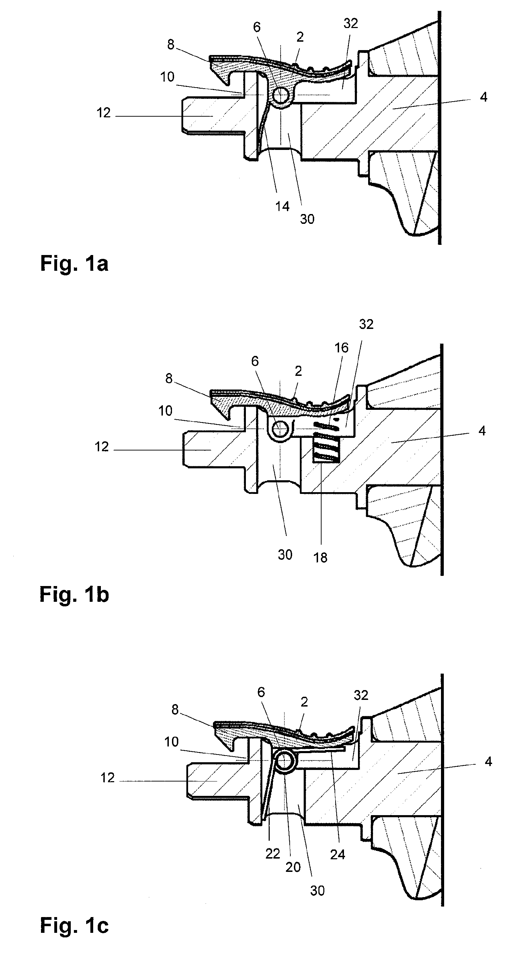

Referring to figures 1a, 1b and 1c, these illustrate portions of three known types of surgical instrument in which a clip 2 is coupled to a handle 4 and arranged to pivot about a pin 6.
A latch 8 of the clip 2 is arranged to move relative to a groove 10 adjacent to a plug 12. The plug 12 is arranged to be received in a socket of a further surgical instrument or component to secure the further surgical instrument 12 to the handle 4. When the plug 12 is engaged in a socket of a further instrument the latch 8 is arranged to engage a groove in the further instrument.

Figure 1a shows the clip 2 being biased under the action of a leaf spring 14 such that latch 8 engages groove 10. The leaf spring 14 is integrally formed with the clip 2. Alternatively, the leaf spring 14 may be integrally formed with the handle 4 or may be a separate component. Similarly, figure 1b shows the clip 2 biased by a coil spring 16 received within a cavity 18 in the handle and acting under compression to bias latch 8 into groove 10. Figure 1c shows the clip 2 biased by a coil spring 20 that acts under torsion to bias clip 2. Coil spring 20 extends around pivot pin 6 and has legs 22, 24 that bear against part of the clip 2 and the handle 4.

More generally, surgical instruments commonly comprise springs arranged to bias movement of a first component relative to a second component. Typically, the springs are stock metal components such as the leaf and coil springs described above. Although such springs function well, they give rise to problems when the instrument requires cleaning (for a reusable instrument). Often additional flushing holes and other features must be incorporated into the instrument to enable the springs to be easily cleaned. For instance, in the surgical instruments illustrated in figures 1a to 1c, a first channel 30 extends from underneath the handle 4, passing through the handle 4 and surrounding the pivot pin 6 to allow for cleaning the pivot of the clip 2. A second channel 32 is provided underneath the clip 2 to allow for cleaning underneath the clip, specifically to provide access for cleaning springs 14, 16, 20. The second channel adds to the complexity and cost of the instrument and can make the instrument harder to understand because users confuse operational features with cleaning features. Furthermore, conventional springs are relatively easy to damage or lose while attempting to clean an instrument, resulting in the instrument either requiring repair or having to be thrown away. Metal springs may also be subject to corrosion.

US-6,358,267 describes a treatment tool for an operation comprising an insertion section inserted into a living body, an operating section including a fixed handle removably connected to the base end side of the insertion section and a movable handle adapted to operate with respect to the fixed handle. An engaging section is arranged at the forward end side of the fixed handle, and an engaging/disengaging member is arranged at the base end of the insertion section to engage the engaging section as required. A biasing member is provided for energizing the disengaging/engaging member toward the position of engagement between the engaging/disengaging member and the engaging section.

It is an object of embodiments of the present invention to obviate or mitigate one or more of the problems associated with the prior art, whether identified herein or elsewhere.

According to a first aspect of the present invention there is provided a surgical instrument comprising: a handle; a clip pivotally coupled to the handle and arranged to move relative to the handle to secure the handle to a further surgical instrument; and a grip secured to the clip and arranged to be manipulated by a user to move the clip relative to the handle; wherein the grip is formed from an elastomeric material and the instrument further comprises an elastomeric spring portion integrally formed with the grip, the spring portion extending from the grip and arranged to bias relative movement between the handle and clip and to bias the clip towards an engaged position in which the further surgical instrument is secured to the handle.

An advantage of the present invention is that because the spring portion comprises part of the grip secured to the second component, this reduces the number of separate components and hence the complexity of the surgical instrument. Furthermore, a spring portion formed from an elastomeric material can have a relatively simple shape having a less complex, and potentially smaller surface area than a convention metallic leaf or coil spring, which reduces the potential for contamination of the instrument. Additionally, as the spring portion is integrally formed with the grip, this makes it easier to clean the instrument as there are fewer cavities for dirt to be trapped within. Consequently, there is less need to provide cleaning channels and other such structures.

The grip may overly and surround a portion of the clip and the clip component is shaped such that the grip interlocks with the clip to secure the grip to the clip.

The clip may comprise at least one indent or hole passing through the clip and the elastomeric material extends into the at least one indent or hole to secure the grip to the clip.

The grip may be shaped to provide an ergonomic grip for a user to manipulate to move the handle and clip relative to one another.

The spring portion may be arranged to be compressed as the clip moves from a first position to a second position relative to the handle so that the clip is biased towards the first position.

The spring portion may be shaped to provide a variable spring response as the handle and clip move relative to one another.

The spring portion may be shaped to provide a non-linear spring response as the handle and clip move relative to one another.

The cross sectional area of the spring portion may vary with distance away from the grip.

According to a second aspect of the present invention there is provided a method of manufacturing a surgical instrument comprising: forming first and second components; moulding an elastomeric material over at least part of the second component to form a grip and an integral spring portion extending from the grip; and coupling the second component to the first component such that the second component can move relative to the first component when the grip is manipulated by a user and the spring portion biases relative movement between the first and second components.

Said step of moulding may form a grip which overlies and surrounds a portion of the second component and the second component is shaped such that the grip interlocks with the second component to secure the grip to the second component.

The method may further comprise forming at least one indent or hole passing into or through the second component such that said step of moulding comprises providing elastomeric material extending into the at least one indent or hole to form the grip and to secure the grip to the second component.

Said step of moulding may comprise: forming a mould surrounding at least part of the grip, the mould being shaped according to the desired shape of the grip including the spring portion; and providing liquid elastomeric material to the mould to form the grip and spring portion.

Embodiments of the present invention will now be described, by way of example only, with reference to the accompanying drawings, in which:
Figures 1a, 1b and 1c illustrate cross sectional views of portions of known surgical instruments incorporating conventional spring components
Figure 2a illustrates a cross sectional view of a portion of a surgical instrument in accordance with an embodiment of the present invention including an elastomeric grip and spring portion with the spring portion at rest;
Figure 2b illustrates the cross sectional view of figure 2a with the spring portion compressed;
Figure 3 is a perspective view of the second component and the grip including the spring portion of figure 2a; and
Figure 4 is a perspective view of a universal handle forming part of a surgical instrument, including the components illustrated in figure 3.

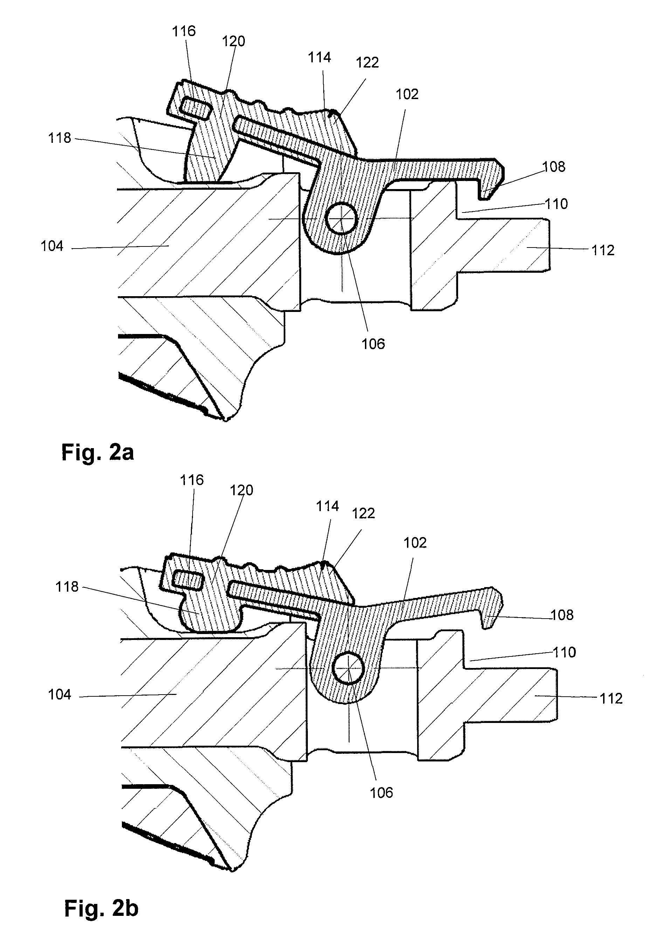

Referring to figure 2a, this illustrates in cross section a portion of a surgical instrument in accordance with an embodiment of the present invention. The surgical instrument of figure 2a is generally similar to the surgical instruments of figures 1a to 1c and comprises a clip 102 coupled to a handle 104 and arranged to pivot about a pin 106. A latch 108 forming part of the clip 102 and arranged to move relative to a groove 110. The handle 104 terminates in a plug 112 arranged to be received in a socket in a further surgical instrument such that the latch 108 can engage or disengage a groove in the further surgical instrument to secure the further surgical instrument to the handle 104.

The surgical instrument of figure 2a differs from that of figures 1a to 1c in that there is no separate spring component. Rather, a grip 114 is formed over and secured to a protruding portion 116 of the clip 102, for instance as a sleeve passing over the protruding portion 116. The grip 114 further comprises an integrally formed spring portion 118. The grip 114 and the spring portion 118 are formed from an elastomeric material. The grip 114 is provided on the clip 102 on the opposite side of pivot 106 to latch 108 such that depressing grip 114 causes the latch 108 to disengage from groove 110 to release an instrument coupled to plug 112.

Spring portion 118 is shown at substantially at rest in figure 2a, although the spring portion 118 remains in contact with the handle 104 and the tip of the spring portion is slightly deformed. Figure 2b illustrates the instrument of figure 2a except that the grip 114 has been depressed towards the handle 104, for instance by being manipulated by the thumb of a user, and the spring portion 118 is compressed. Compression of the elastomeric spring portion 118 requires the resistance of the spring portion 118 to compression to be overcome by the force applied to the grip 114 by the user deforming the spring portion 118. When the grip 114 is released the spring portion 118 reverts to its original shape thereby biasing the clip 102 so that latch 108 is in the engaged position.

The spring portion 118 is integrally formed with the grip 114 and may be formed using a conventional manufacturing process such as injection moulding the elastomeric material. For instance, an elastomeric material such as silicon is injected into a mould surrounding the end of clip 102 so that the material forms around the clip 102 and secures the grip 114 and the spring portion 118 to the clip 102. In order to ensure that the grip 114, and hence the spring portion 118, cannot easily be removed from the clip 102, the clip 102 may be shaped or textured to ensure that the elastomeric material is firmly keyed into the clip. For instance, a hole 120may be provided passing through the clip 102, or there may be an indent in the surface of the clip 102, into which the elastomeric material flows during moulding. Once set, the grip 114 cannot be simply pulled from the end of the clip 102.

Alternative manufacturing techniques will be well known the appropriately skilled person. For instance, compression or transfer moulding may be used in place of injection moulding.

As discussed above, the spring portion 118 biases the grip 114 away from the handle 104. Figures 2a and 2b illustrate the spring portion 118 as a generally conical structure. However, it will be appreciated that the spring portion 118 may comprise any shape. Consequently, the spring portion may be profiled to provide any required spring response, which may be linear or non-linear. The spring force is generally proportional to the cross section of the spring portion under compression. The spring portion 118 illustrated in figures 2a and 2b narrows away from the clip 102. Consequently when the spring portion 118 presses against the handle 104 the narrowest portions will undergo the greatest degree of compression first and so the spring force starts low and increases as the grip portion 114 moves towards the handle 104. It will be readily apparent to the appropriately skilled person that the shape of the spring portion 118 may be changed in order to provide any response. For instance, the spring portion may be stepped when viewed in the cross sectional view of figures 2a and 2b so that there is at least one discontinuity in the spring force as the grip 114 moves towards the handle 104. Alternatively, two or more separate spring portions may be provided upon the grip 114 having different heights such that the spring portions are progressively engaged as the grip moves towards the handle. Other options will be readily apparent according to the required spring response.

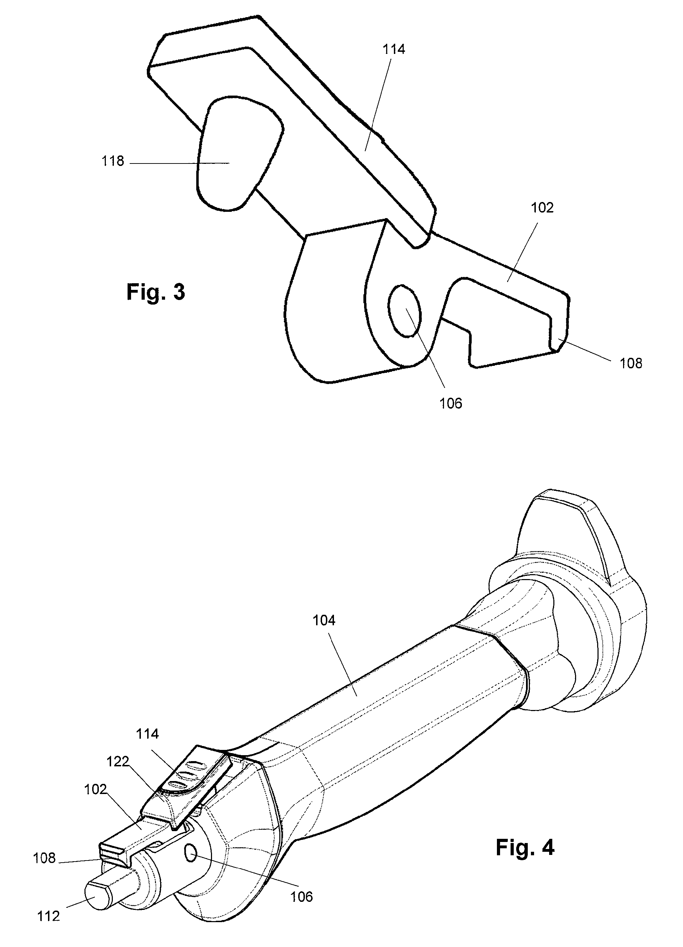

Figure 3 illustrates the clip 102 removed from the handle 104 in a perspective view. It can be readily be seen that the grip 114 substantially surrounds at least the protruding portion of the clip 102. The spring portion 118 depends from the underside of the grip 114 and is a generally rounded conical structure, though as discussed above can be of any shape.

The grip 114 provides a tactile grip for the user to engage the clip 102 due to the inherent properties of elastomeric materials. Advantageously, this makes is easier to operate the clip, for instance if covered in blood during an operation. The grip may be made easier still to manipulate by providing a textured surface, as visible in the cross sectional views of figures 2a and 2b and in the perspective view of figure 4. The grip 114 also has a raised end portion 122 to reduce the risk of the user's thumb sliding from the end of the grip 114. Further options for the shape of the grip 114 will be readily apparent to the skilled person according to the particular requirements for the user to manipulate part of a surgical instrument. Advantageously, for surgical instruments where there is a recognised need to provide a tactile grip, such as a thumb grip, the spring portion may be provided as part of the same manufacturing step at minimal further cost, certainly less than the cost of providing a separate spring component.

The present invention has been described above in connection with a spring portion integrally formed with a grip attached to a clip coupled to a handle. More generally, the spring portion can also be used with any surgical instrument, or portion of a surgical instrument, having first and second components coupled together and arranged to move relative to one another where there is a requirement to provide a spring to bias movement of the components and where it is desirable to form the spring integrally with a grip for manipulating one or other of the components. For instance, the spring portion could be used to provide a spring-loaded instrument adjustment mechanism forming part of a surgical instrument.

Components of the instrument other than the grip and the spring portion may be formed from any suitable materials known in the art, for instance a metal such as stainless steel. The elastomeric grip and spring portion may be coloured to contrast with the remainder of the instrument, or any required colour scheme. Advantageously, this draws the user's attention to the grip making it readily apparent that it is a part of the instrument intended to be operated, thereby making the instrument easier to interpret.

The embodiments of the invention described above relate to an integral elastomeric grip and spring portion in which the spring portion acts under compression to bias relative movement of first and second components of a surgical instrument. However, it will be readily apparent that alternatively the elastomeric spring portion may be arranged to act under extension or shear in order to bias the movement of the two components. In order for the spring portion to act under extension or shear the spring portion may be coupled permanently or temporarily to both components of the instrument.

Further applications of, and modifications to, the present invention will be readily apparent to the appropriately skilled person from the teaching herein without departing from the scope of the appended claims.

## Claims

1. A surgical instrument comprising:
a handle (104);
a clip (102) pivotally coupled to the handle and arranged to move relative to the handle to secure the handle to a further surgical instrument; and
a grip (114) secured to the clip and arranged to be manipulated by a user to move the clip relative to the handle; wherein the grip is formed from an elastomeric material and the instrument further comprises an elastomeric spring portion (118) integrally formed with the grip, the spring portion extending from the grip and arranged to bias relative movement between the handle and clip and to bias the clip towards an engaged position in which the further surgical instrument is secured to the handle.

2. A surgical instrument according to claim 1, wherein the grip (114) overlies and surrounds a portion of the clip (102) and the clip is shaped such that the grip interlocks with the clip to secure the grip to the clip.

3. A surgical instrument according to claim 2, wherein the clip (102) comprises at least one indent or hole (120) passing through the clip and the elastomeric material extends into the at least one indent or hole to secure the grip to the clip.

4. A surgical instrument according to any preceding claim, wherein the grip (114) is shaped to provide an ergonomic grip for a user to manipulate to move the handle (104) and clip (102) relative to one another.

5. A surgical instrument according to any preceding claim, wherein the spring portion (118) is arranged to be compressed as the clip (102) moves from a first position to a second position relative to the handle (104) so that the clip is biased towards the first position.

6. A surgical instrument according to any preceding claim, wherein the spring portion (118) is shaped to provide a variable spring response as the handle (104) and clip (102) move relative to one another.

7. A surgical instrument according to claim 6, wherein the spring portion (118) is shaped to provide a non-linear spring response as the handle (104) and clip (102) move relative to one another.

8. A surgical instrument according to claim 6 or claim 7, wherein the cross sectional area of the spring portion (118) varies with distance away from the grip.

9. A method of manufacturing a surgical instrument comprising:
forming first and second components;
moulding an elastomeric material over at least part of the second component to form a grip (114) and an integral spring portion (118) extending from the grip; and
coupling the second component to the first component such that the second component can move relative to the first component when the grip is manipulated by a user and the spring portion (118) biases relative movement between the first and second components.

10. A method according to claim 9, wherein said step of moulding forms a grip which overlies and surrounds a portion of the second component and the second component is shaped such that the grip interlocks with the second component to secure the grip to the second component.

11. A method according to claim 10, further comprising forming at least one indent or hole (120) passing into or through the second component such that said step of moulding comprises providing elastomeric material extending into the at least one indent or hole to form the grip and to secure the grip to the second component.

12. A method according to any one of claims 9 to 11, wherein said step of moulding comprises:
forming a mould surrounding at least part of the grip, the mould being shaped according to the desired shape of the grip including the spring portion; and
providing liquid elastomeric material to the mould to form the grip and spring portion.

## Patentansprüche

1. Chirurgisches Instrument umfassend:
einen Handgriff (104);
eine Klemme (102), die schwenkbar an dem Handgriff gekoppelt ist und angeordnet ist zur Bewegung relativ zum Handgriff, um den Handgriff an einem weiteren chirurgischen Instrument zu befestigen; und
einen Griff (114), der an der Klemme befestigt ist und angeordnet ist, um von einem Benutzer gehandhabt zu werden, um die Klemme relativ zum Handgriff zu bewegen; wobei der Griff aus einem elastomeren Material gebildet ist und das Instrument weiterhin einen elastomeren Federabschnitt (118) umfasst, der integral mit dem Griff gebildet ist, wobei der Federabschnitt sich von dem Griff erstreckt und angeordnet ist zum Vorspannen der relativen Bewegung zwischen dem Handgriff und der Klemme und zum Vorspannen der Klemme hin zu einer in Eingriffsposition, in der das weitere chirurgische Instrument an dem Handgriff befestigt ist.

2. Chirurgisches Instrument nach Anspruch 1, wobei der Griff (114) einen Abschnitt der Klemme (102) überlagert und umgibt und die Klemme derart gestaltet ist, dass der Griff mit der Klemme ineinandergreift, um den Griff an die Klemme zu befestigen.

3. Chirurgisches Instrument nach Anspruch 2, wobei die Klemme (102) mindestens eine Aussparung oder Öffnung (120) umfasst, die durch die Klemme führt und das elastomere Material sich in die mindestens eine Aussparung oder Öffnung zur Befestigung des Griffs an die Klemme erstreckt.

4. Chirurgisches Instrument nach einem vorangegangenen Anspruch, wobei der Griff (114) zur Bereitstellung eines ergonomischen Griffs für einen Benutzer zur Handhabung zur Bewegung des Handgriffs (104) und der Klemme (102) relativ zueinander gestaltet ist.

5. Chirurgisches Instrument nach irgendeinem vorangegangenen Anspruch, wobei der Federabschnitt (118) angeordnet ist, um zusammengedrückt zu werden, wenn die Klemme (102) sich von einer ersten Position zu einer zweiten Position relativ zu dem Handgriff (104) bewegt, sodass die Klammer zu der ersten Position hin vorgespannt wird.

6. Chirurgisches Instrument nach irgendeinem vorangegangenen Anspruch, wobei der Federabschnitt (118) zur Bereitstellung einer variablen Feder-Antwort gestaltet ist, wenn der Handgriff (104) und die Klemme (102) sich relativ zueinander bewegen.

7. Chirurgisches Instrument nach Anspruch 6, wobei der Federabschnitt (118) zur Bereitstellung einer nichtlinearen Feder-Antwort gestaltet ist, wenn der Handgriff (104) und die Klemme (102) sich relativ zueinander bewegen.

8. Chirurgisches Instrument nach Anspruch 6 oder Anspruch 7, wobei der Querschnittsbereich des Federabschnitts (118) mit einem Abstand weg von dem Griff variiert.

9. Verfahren zur Herstellung eines chirurgischen Instruments, umfassend:
Bilden einer ersten und zweiten Komponente;
Formen eines elastomeren Materials über mindestens einem Teil der zweiten Komponente zum Bilden eines Griffs (114) und eines integralen Federabschnitts (118), der sich von dem Griff erstreckt; und
Koppeln der zweiten Komponente an die erste Komponente, sodass die zweite Komponente relativ zu der ersten Komponente bewegt werden kann, wenn der Griff von einem Benutzer gehandhabt wird und der Federabschnitt (118) eine relative Bewegung zwischen der ersten und zweiten Komponente vorspannt.

10. Verfahren nach Anspruch 9, wobei der Schritt des Formens einen Griff bildet, welcher einen Abschnitt der zweiten Komponente überlagert und umgibt und die zweite Komponente so gestaltet ist, dass der Griff mit der zweiten Komponente zur Befestigung des Griffs an der zweiten Komponente verriegelt.

11. Verfahren nach Anspruch 10, zudem umfassend Ausbilden mindestens einer Aussparung oder Öffnung (120), die in die zweite Komponente oder durch sie hindurch führt, sodass der Schritt des Formens ein Bereitstellen von elastomerem Material umfasst, das sich in die mindestens eine Aussparung oder Öffnung zur Bildung des Griffs und zur Befestigung des Griffs an der zweiten Komponente erstreckt.

12. Verfahren nach irgendeinem der Ansprüche 9 bis 11, wobei der Schritt des Formens umfasst:
Bilden einer Form, die mindestens einen Teil des Griffs umgibt, wobei die Form gemäß der gewünschten Gestalt des Griffs, einschließlich des Federabschnitts, ausgebildet ist; und
Bereitstellen von flüssigem elastomerem Material für die Form, um den Griff und den Federabschnitt zu bilden.

## Revendications

1. Instrument chirurgical comprenant :
un manche (104) ;
une agrafe (102) couplée de manière pivotante au manche et agencée pour se déplacer par rapport au manche afin de fixer le manche à un instrument chirurgical supplémentaire ; et
une poignée (114) fixée à l'agrafe et agencée pour être manipulée par un utilisateur afin de déplacer l'agrafe par rapport au manche ; dans lequel la poignée est constituée d'un matériau élastomère et l'instrument comprend en outre une partie de ressort élastomère (118) mise en forme solidairement avec la poignée, la partie de ressort s'étendant à partir de la poignée et agencée pour solliciter un mouvement relatif entre la poignée et l'agrafe et pour solliciter l'agrafe vers une position enclenchée dans laquelle l'instrument chirurgical supplémentaire est fixé au manche.

2. Instrument chirurgical selon la revendication 1, dans lequel la poignée (114) recouvre et entoure une partie de l'agrafe (102) et l'agrafe est mise en forme de sorte que la poignée s'emboîte avec l'agrafe afin de fixer la poignée à l'agrafe.

3. Instrument chirurgical selon la revendication 2, dans lequel l'agrafe (102) comprend au moins un renfoncement ou trou (120) traversant l'agrafe et le matériau élastomère s'étend dans le au moins un renfoncement ou trou pour fixer la poignée à l'agrafe.

4. Instrument chirurgical selon l'une quelconque des revendications précédentes, dans lequel la poignée (114) est mise en forme pour fournir une poignée ergonomique destinée à ce qu'un utilisateur manipule le manche (104) et l'agrafe (102) afin qu'ils se déplacent l'un par rapport à l'autre.

5. Instrument chirurgical selon l'une quelconque des revendications précédentes, dans lequel la partie de ressort (118) est agencée pour être comprimée à mesure que l'agrafe (102) passe d'une première position à une seconde position par rapport au manche (104) de sorte que l'agrafe soit sollicitée vers la première position.

6. Instrument chirurgical selon l'une quelconque des revendications précédentes, dans lequel la partie de ressort (118) est mise en forme pour fournir une réponse de ressort variable à mesure que le manche (104) et l'agrafe (102) se déplacent l'un par rapport à l'autre.

7. Instrument chirurgical selon la revendication 6, dans lequel la partie de ressort (118) est mise en forme pour fournir une réponse de ressort non linéaire à mesure que le manche (104) et l'agrafe (102) se déplacent l'un par rapport à l'autre.

8. Instrument chirurgical selon la revendication 6 ou la revendication 7, dans lequel l'aire en coupe de la partie de ressort (118) varie avec la distance en s'éloignant de la poignée.

9. Méthode de fabrication d'un instrument chirurgical comprenant :
la formation de premier et second composants ;
le moulage d'un matériau élastomère sur au moins une partie du second composant pour former une poignée (114) et une partie de ressort solidaire (118) s'étendant à partir de la poignée, et
le couplage du second composant au premier composant de sorte que le second composant puisse se déplacer par rapport au premier composant lorsque la poignée est manipulée par un utilisateur et la partie de ressort (118) sollicite un déplacement relatif entre les premier et second composants.

10. Méthode selon la revendication 9, dans laquelle ladite étape de moulage forme une poignée qui recouvre et entoure une partie du second composant et le second composant est mis en forme de sorte que la poignée s'emboîte avec le second composant pour fixer la poignée au second composant.

11. Méthode selon la revendication 10, comprenant en outre la formation d'au moins un renfoncement ou trou (120) passant dans ou traversant le second composant de sorte que ladite étape de moulage comprend la fourniture d'un matériau élastomère s'étendant dans le au moins un renfoncement ou trou afin de former la poignée et de fixer la poignée au second composant.

12. Méthode selon l'une quelconque des revendications 9 à 11, dans laquelle ladite étape de moulage comprend :
la formation d'un moule entourant au moins une partie de la poignée, le moule étant mis en forme selon la forme souhaitée de la poignée comprenant la poignée de ressort ; et
la fourniture d'un matériau élastomère liquide au moule afin de former la poignée et la partie de ressort.
